# FASCICULE DE BREVET EUROPEEN

(11) **EP 0 897 528 B1**
(45) Date de publication et mention de la délivrance du brevet: **13.03.2002**
(21) Numéro de dépôt: 97924073.6
(22) Date de dépôt: 12.05.1997
(51) Int. Cl.: G01L 9/08

(54) **DISPOSITIF DE MESURE DE LA PRESSION D'UN LIQUIDE CIRCULANT DANS UNE TUBULURE VERS OU HORS DU CORPS HUMAIN**
VORRICHTUNG ZUR MESSUNG DES DRUCKES EINER FLÜSSIGKEIT, DIE MITTELS EINES RÖHRCHENS IN DEN ODER AUS DEM MENSCHLICHEN KÖRPER FLIESST
DEVICE FOR MEASURING THE PRESSURE OF A FLUID FLOWING INTO OR OUT OF THE HUMAN BODY THROUGH A TUBING

(30) Priorité: 10.05.1996 FR 9605872
(43) Date de publication de la demande: 24.02.1999
(73) Titulaire: CORNEAL INDUSTRIE, 74370 Pringy (FR)
(72) Inventeur: CROZAFON, Philippe, F-06000 Nice (FR); BOS, Gilles, F-74330 La Balme de Sillingy (FR); ORTUNO, Angel, F-74330 Choisy (FR)
(74) Mandataire: Dronne, Guy
(86) Numéro de dépôt international: FR9700834
(87) Numéro de publication internationale: WO9743615

(56) Documents cités:
- EP-A- 0 092 827
- US-A- 3 863 504
- PATENT ABSTRACTS OF JAPAN vol. 095, no. 011, 26 Décembre 1995 & JP 07 198521 A (TASUKO JAPAN KK;OTHERS: 01), 1 Août 1995,
- PATENT ABSTRACTS OF JAPAN vol. 095, no. 009, 31 Octobre 1995 & JP 07 155857 A (SHOWA ALUM CORP), 20 Juin 1995,
- PATENT ABSTRACTS OF JAPAN vol. 008, no. 100 (M-295), 11 Mai 1984 & JP 59 012254 A (SANYO DENKI KK;OTHERS: 01), 21 Janvier 1984,

## Description

La présente invention a pour objet un dispositif de mesure de la pression d'un liquide circulant dans une tubulure et extrait du corps humain.

De façon plus précise, l'invention concerne un dispositif qui permet, en permanence, de mesurer la pression d'un liquide qui circule dans une tubulure raccordée à une partie du corps humain, cette tubulure servant à l'extraction hors du corps humain de ce liquide, la pression étant alors bien sûr négative par rapport à la pression atmosphérique, l'aspiration étant également réalisée à l'aide d'une pompe.

Un tel problème se présente notamment mais bien sûr non exclusivement dans la mise en oeuvre de la technique dite de phaco-émulsification. Cette technique, qui concerne la chirurgie oculaire, consiste à réaliser l'ablation du cristallin par fragmentation de celui-ci à l'aide d'un émetteur d'ultrasons. Les fragments de cristallin ainsi obtenus doivent être extraits à l'aide d'une canule de l'oeil et, plus précisément, du sac capsulaire qui contient le cristallin. Cette extraction est réalisée à l'aide d'une tubulure reliée à une pompe d'extraction qui permet ainsi d'extraire les fragments de cristallin noyés dans un liquide qui est du type sérum physiologique. Simultanément, bien sûr, à l'aide d'une autre canule, on injecte dans l'oeil et, plus précisément, dans le sac capsulaire, un liquide physiologique de synthèse pour remplacer au moins temporairement le cristallin extrait, cette injection étant le plus souvent réalisée par simple effet de gravité.

Dans ce type d'opération, il est extrêmement important que le volume de matériau liquide et solide contenu à chaque instant dans le cristallin soit sensiblement constant afin de maintenir sensiblement constante la forme de la chambre antérieure. En effet, en particulier en cas de diminution importante et soudaine de ce volume, cela pourrait entraîner des altérations importantes et irréversibles de l'oeil et notamment de la rétine par décollement de celle-ci, du fait d'une chute brutale de la pression à laquelle elle est soumise par l'humeur vitrée.

Or, dans l'opération de phaco-émulsification, et, plus précisément, lors de l'opération d'aspiration des fragments du cristallin, de telles variations de pression soudaines peuvent se présenter lorsque, par exemple, un fragment du cristallin obture temporairement la canule d'extraction. En effet, dans ce cas, lorsque l'obturation par le fragment du cristallin cesse, du fait de la dépression créée dans la tubulure, on a une aspiration avec un débit très important de liquide qui n'est pas compensé pas l'alimentation en liquide de substitution.

Il faut rappeler de plus que, du fait de l'extraction la pression "normale" du liquide est inférieure à la pression atmosphérique et est donc une dépression. Dans les conditions opératoires habituelles, la dépression par rapport à la pression atmosphérique est de l'ordre de 50 à 500 mm de mercure et plus souvent comprise entre 150 et 350 mm de mercure.

Dans ce cas, comme dans d'autres utilisations, il est important de pouvoir contrôler en permanence, de façon précise et fiable, et avec une constante de temps très faible, la pression régnant effectivement dans la tubulure, qu'elle soit d'aspiration ou d'injection.

Or, en particulier dans les techniques de phaco-émulsification, le contrôle de la pression dans la tubulure est réalisée par un élément de conduite montée en dérivation sur la tubulure principale qui est raccordée à un capteur de pression. Ce tuyau de dérivation est rempli d'air afin de protéger la membrane sensible du capteur vis-à-vis de l'agression qui pourrait naître du liquide circulant dans la tubulure. En cas de variation soudaine de pression, l'air emprisonné dans cette tubulure, qui est bien sûr beaucoup plus compressible que le liquide circulant dans la tubulure, peut entraîner d'une part une altération sensible de la valeur de la pression mesurée et surtout un retard important entre la variation effective de pression dans la tubulure et sa détection par le capteur de pression. Ces erreurs de mesure peuvent, comme on l'a expliqué précédemment, entraîner des conséquences très dommageables lors de l'intervention, notamment dans le cas de la phaco-émulsification.

Un objet de la présente invention est de remédier à cet inconvénient en fournissant un dispositif de mesure de pression qui peut être monté sur une tubulure servant à l'aspiration d'un liquide hors du corps humain, et qui permette de mesurer à chaque instant, avec une grande précision et une constante de temps très réduite, la pression effective du liquide circulant dans la tubulure.

Pour atteindre ce but, selon l'invention, le dispositif de mesure de la pression du liquide circulant dans une tubulure de mise en circulation d'un liquide hors du corps humain se caractérise en ce qu'il comprend :
des moyens formant tubulure présentant un alésage axial dont la face interne de la paroi constitue sensiblement une solution de continuité de la tubulure, lesdits moyens comportant un orifice traversant la paroi et débouchant dans la face interne, une première extrémité raccordée à une première portion de ladite tubulure et une deuxième extrémité raccordée à une deuxième portion de la tubulure, et
un capteur de pression pour mesurer la pression dudit liquide dans la tubulure, ledit capteur présentant une surface soumise à la pression dudit liquide, au moins une partie dudit capteur étant montée de manière étanche dans ledit orifice, de telle manière que ladite surface sensible constitue sensiblement une solution de continuité de la face interne de la paroi des moyens formant tubulure.

Selon un mode préféré de mise en oeuvre de l'invention, le dispositif comprend
un corps allongé présentant une première et une deuxième extrémité dans lequel est ménagé un alésage axial débouchant dans lesdites première et deuxième extrémités et limité par une paroi interne, chaque extrémité présentant des moyens de raccordement à ladite tubulure, ledit corps présentant de plus un évidement débouchant dans ledit alésage axial entre ses première et deuxième extrémités; et
un capteur de pression monté dans ledit évidement, ledit capteur présentant une surface soumise à la pression dont la forme est sensiblement une solution de continuité de la paroi interne de l'alésage au niveau dudit évidement.

On comprend que, grâce à cette disposition intégrée du capteur de pression, la face du capteur soumise à la pression est sensiblement dans la continuité de l'élément de tubulure défini par le corps du dispositif de mesure de pression. Ainsi, on évite toute accumulation accidentelle d'air ou de tout autre gaz qui viendrait perturber dans le temps et en grandeur la mesure de la pression régnant effectivement dans la tubulure.

Dans le présent texte, par "sensiblement une solution de continuité" signifie que la surface sensible du capteur de pression n'est pas au sens strict une portion de la surface correspondant à la paroi interne des moyens formant tubulure, mais que cette surface sensible ne crée qu'un volume mort très réduit par rapport à la surface correspondant à la paroi interne. Ce volume est de préférence inférieur à 10 mm³ et de préférence encore inférieur à 5 mm³.

D'autres caractéristiques et avantages de l'invention apparaîtront mieux à la lecture de la description qui suit d'un mode de réalisation de l'invention donné à titre d'exemple non limitatif.

La description se réfère aux figures annexées sur lesquelles :
- la figure 1 est une vue en coupe longitudinale de l'ensemble du dispositif de mesure de pression selon un premier mode de réalisation;
- la figure 2 est une vue en coupe selon la ligne II-II de la figure 1;
- la figure 3 est une vue de face du capteur de pression proprement dit;
- la figure 4 est une vue de côté du capteur de pression de la figure 3;
- la figure 5 est une vue de dessus du capteur de pression; et
- la figure 6 est une vue partielle en coupe axiale d'un deuxième mode de réalisation du dispositif de mesure de pression.

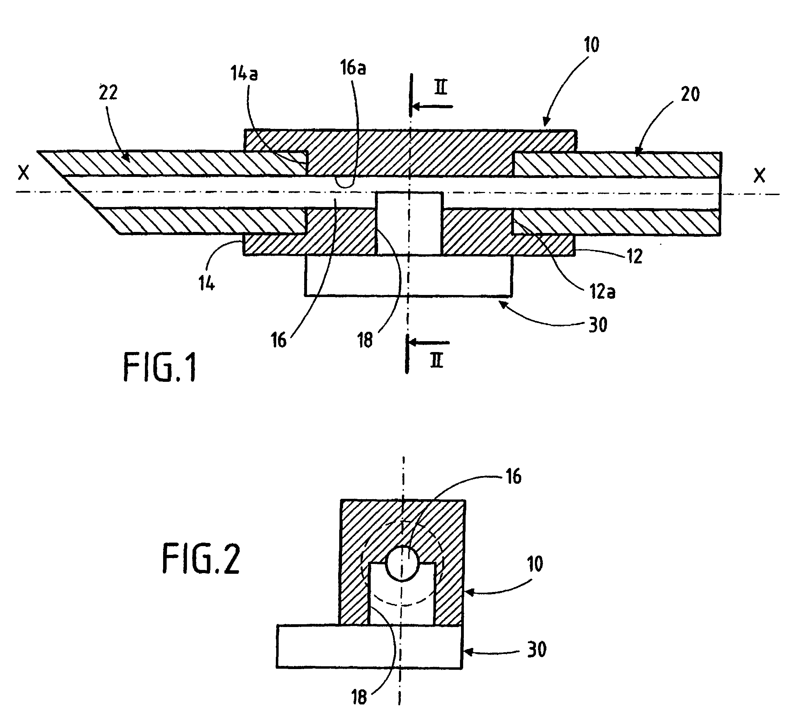

En se référant tout d'abord aux figures 1 et 2, on va décrire l'ensemble du dispositif de mesure de pression. Selon un mode préféré de mise en oeuvre, ce dispositif est essentiellement constitué par un corps de forme allongée 10 présentant deux extrémités 12 et 14. Dans le corps 10, est ménagé un alésage axial de préférence de forme cylindrique 16, cet alésage axial 16 débouchant dans les extrémités 12 et 14 du corps 10. Le corps 10 est par exemple de forme à section droite rectangulaire. Le corps 10 est également muni d'un évidement 18 dont la direction principale est disposée orthogonalement à l'axe XX' de l'alésage axial 16. L'évidement 18 débouche dans l'alésage axial 16.

De préférence, les extrémités 12 et 14 du corps 10 du dispositif comportent une portion en retrait respectivement 12a et 14a permettant le raccordement étanche du corps 10 aux portions de tubulure amont 20 et aval 22, dans lesquelles circule le liquide qui doit être extrait d'une partie du corps humain, notamment de l'oeil dans le cas où le dispositif de mesure de pression sert à la mise en oeuvre d'une opération de phaco-émulsification. Une des portions de tubulure est raccordée à la pompe d'aspiration et l'autre portion est raccordée à une canule.

De préférence, le corps 10 du dispositif est réalisé par moulage d'un matériau plastique biocompatible et rigide tel que du PMMA ou autre matériau analogue.

Le dispositif de mesure de pression comporte également un capteur de pression différentiel 30 qui est monté sur le corps 10 et qui comporte, comme on l'expliquera, une extension pénétrant dans l'évidement 18.

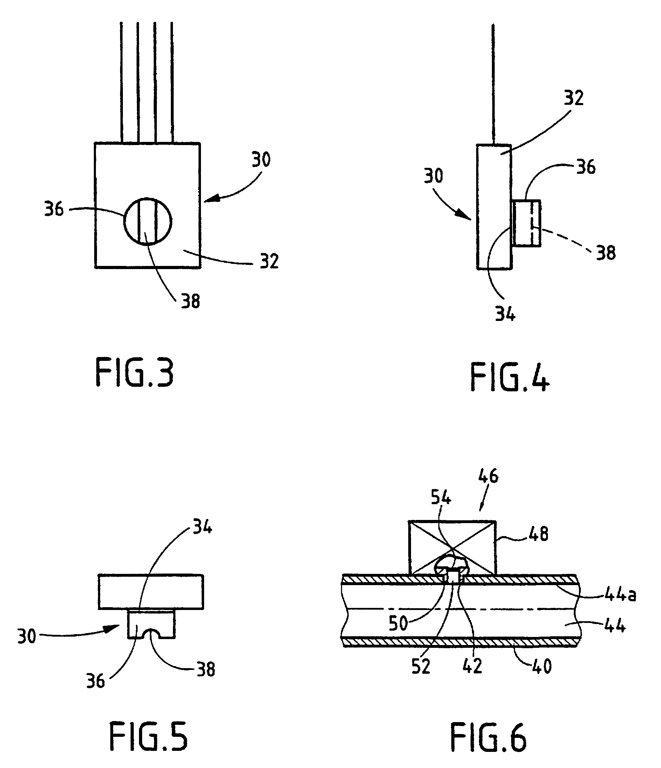

Le capteur de pression différentiel 30, comme le montre mieux la figure 4, comprend un boîtier de traitement de signaux électriques et de connexion 32 et une surface sensible 34 en matériau piézo-électrique. En regard de la surface sensible 34, est prévu un bloc 36 réalisé en un gel, ce bloc pénétrant de façon étanche dans l'évidement 18 du corps 10. En outre, le bloc 36 présente une surface qui définit en partie une portion de surface cylindrique 38, de même rayon que l'alésage axial, comme le montrent les figures 4 et 5. Cette portion de surface cylindrique 38 constitue une solution de continuité de la paroi interne 16a de l'évidement axial 16 ménagé dans le corps 10.

Il va de soi que la section droite de l'alésage 16 pourrait ne pas être circulaire. Dans ce cas, la surface 38 du bloc 36 aurait une forme adaptée à cette section droite.

Grâce à cette disposition, on évite tout singularité dans l'alésage axial 16 au niveau du capteur de pression. En conséquence, on évite ainsi tout risque d'accumulation d'air lors de la circulation du liquide dans la tubulure et donc dans l'alésage axial 16.

Il faut également préciser que le bloc 34 est réalisé à l'aide d'un gel non compressible qui transmet intégralement la pression reçue sur la surface cylindrique 38 à la surface sensible piézo-électrique 34 du capteur de pression différentiel.

On pourrait bien sûr utiliser un autre élément convertisseur sensible à la pression qu'une membrane piézo-électrique. Cependant, un bloc de matériau incompressible sera interposé entre cet élément sensible et le liquide dont on veut mesurer la pression.

On comprend qu'ainsi, la mesure de pression réalisée par le capteur de pression 30 correspond effectivement à la pression régnant dans l'alésage 16, c'est-à-dire dans la tubulure dans laquelle circule le liquide et cette pression mesurée correspond à chaque instant à la pression effective dans la tubulure. En effet, il n'y a pas interposition d'un matériau compressible entre le débit de liquide et la surface sensible du capteur de pression.

On comprend qu'ainsi, le dispositif de mesure de pression selon l'invention résout exactement le problème posé puisque, par sa géométrie, il permet de mesurer la pression effective du liquide circulant dans la tubulure 20, 22, à chaque instant, sans qu'il apparaisse une constante de temps significative dans cette mesure. Il est ainsi possible, à partir des indications fournies par le capteur de pression, de commander effectivement la pompe d'extraction du liquide, dans le cas de la phaco-émulsification, en fonction de cette pression et en particulier de contrôler de façon très précise, grâce à la mesure de la pression dans la tubulure, le débit du liquide dans le cas où des débris du cristallin viendraient à obturer la canule montée à l'extrémité de la portion de tubulure amont 20, et donc malgré des risques de variations brutales de la pression.

La figure 6 montre un autre mode de réalisation du dispositif de mesure de pression. On retrouve les moyens formant tubulure 40 dans la paroi de laquelle est percé un orifice radial 42 débouchant dans l'alésage axial 44. La paroi interne 44a de celui-ci est sensiblement cylindrique.

Le capteur 46 comprend un corps 48 comportant l'élément convertisseur et un prolongement constitué par une jupe cylindrique 50. Le diamètre externe de la jupe 50 est égal au diamètre de l'orifice 42 pour assurer la fixation étanche du capteur 46 sur les moyens formant tubulure 40. Au fond de la cavité cylindrique 52 limitée par la jupe 48, est montée la surface sensible 54 du capteur qui est soumise à la pression. Cette surface sensible est réalisée en un matériau incompressible qui transmet la pression du liquide à l'élément convertisseur du capteur.

La cavité 52 a des dimensions très réduites de telle manière que le volume mort qu'elle définit par rapport à la paroi interne 44a des moyens formant tubulure soit également très réduit. Dans l'exemple décrit, ce volume est de l'ordre de 4 mm³. Dans tous les cas, on s'arrangera pour que ce volume mort soit inférieur à 10 mm³. Ainsi, même si de l'air y était emprisonné sous l'effet de l'aspiration, le volume d'air est suffisamment réduit pour ne pas risquer d'introduire de distorsions significatives dans la mesure de la pression du liquide dans la tubulure.

## Revendications

1. Dispositif de mesure de la pression d'un liquide circulant dans une tubulure et extrait du corps humain, **caractérisé en ce qu'**il comprend :
- des moyens formant tubulure présentant un alésage axial dont la face interne de la paroi constitue sensiblement une solution de continuité de la tubulure, lesdits moyens comportant un orifice traversant la paroi et débouchant dans la face interne, une première extrémité raccordée à une première portion de ladite tubulure et une deuxième extrémité raccordée à une deuxième portion de la tubulure, et
- un capteur de pression pour mesurer la pression dudit liquide dans la tubulure, ledit capteur présentant une surface soumise à la pression dudit liquide, au moins une partie dudit capteur étant montée de manière étanche dans ledit orifice, de telle manière que ladite surface sensible constitue sensiblement une solution de continuité de la face interne de la paroi des moyens formant tubulure.

2. Dispositif selon la revendication 1, **caractérisé en ce que** le volume mort limité par la surface sensible du capteur et par la surface sur laquelle est inscrite la face interne de la paroi des moyens formant tubulure est inférieur à 10 mm³.

3. Dispositif selon la revendication 2, **caractérisé en ce que** ledit capteur de pression comprend un corps extérieur aux moyens formant tubulure et une extension en forme de jupe cylindrique apte à coopérer de façon étanche avec ledit orifice, la surface sensible à la pression étant disposée à l'extrémité interne de la jupe, la jupe ayant une longueur axiale sensiblement égale à l'épaisseur de la paroi des moyens formant tubulure.

4. Dispositif selon l'une quelconque des revendications 1 à 3, **caractérisé en ce que** ladite surface sensible à la pression est constituée par un matériau incompressible apte à transmettre à l'élément convertisseur du capteur la pression appliquée à ladite surface.

5. Application du dispositif selon l'une quelconque des revendications 1 à 4 à la réalisation d'un appareil de phako-émulsification, **caractérisée** en qu'une des portions de tubulure est raccordée à une pompe d'aspiration et l'autre portion de tubulure est raccordée à une canule.

## Patentansprüche

1. Vorrichtung zur Messung des Druckes einer Flüssigkeit, die in einem Röhrchen fließt und aus dem menschlichen Körper extrahiert wird, **dadurch gekennzeichnet, dass** sie aufweist:
- ein Röhrchen bildende Mittel mit einer axialen Bohrung, deren Innenseite der Seitenwand im wesentlichen eine offene Stelle des Röhrchens bildet, wobei die Mittel eine Öffnung aufweisen, die die Seitenwand durchquert und in der Innenseite endet, wobei ein erstes Ende mit einem ersten Abschnitt des Röhrchens verbunden ist und ein zweites Ende mit einem zweiten Abschnitt des Röhrchens verbunden ist, und
- einen Druckfühler zum Messen des Druckes der Flüssigkeit in dem Röhrchen, wobei der Fühler eine Oberfläche aufweist, die dem Druck der Flüssigkeit ausgesetzt ist, wobei wenigstens ein Teil des Fühlers dicht in der Öffnung befestigt ist, derart, dass die empfindliche Oberfläche im wesentlichen eine offene Stelle der Innenseite der Seitenwand der ein Röhrchen bildenden Mittel ist.

2. Vorrichtung nach Anspruch 1, **dadurch gekennzeichnet, dass** das tote Volumen, das durch die empfindliche Oberfläche des Fühlers und durch die Oberfläche, auf der die Innenseite der Seitenwand der ein Röhrchen bildenden Mittel einbeschrieben ist, begrenzt ist, kleiner als 10 mm³ ist.

3. Vorrichtung nach Anspruch 2, **dadurch gekennzeichnet, dass** der Druckfühler einen Außenkörper mit ein Röhrchen bildenden Mitteln und eine Erweiterung in Form eines zylindrischen Mantels aufweist, die dicht mit der Öffnung zusammenwirken kann, wobei die druckempfindliche Oberfläche am inneren Ende des Mantels angeordnet ist, wobei der Mantel eine axiale Länge aufweist, die im wesentlichen gleich der Dicke der Seitenwand der ein Röhrchen bildenden Mittel ist.

4. Vorrichtung nach einem der Ansprüche 1 bis 3, **dadurch gekennzeichnet, dass** die druckempfindliche Oberfläche aus einem nicht komprimierbaren Material gebildet ist, das dem Konverterelement des Fühlers den an der Oberfläche applizierten Druck übertragen kann.

5. Anwendung der Vorrichtung nach einem der Ansprüche 1 bis 4 bei der Herstellung eines Geräts zur Phakoemulsifikation, **dadurch gekennzeichnet, dass** einer der Röhrchenabschnitte mit einer Absaugpumpe verbunden ist und der andere Röhrchenabschnitt mit einer Kanüle verbunden ist.

## Claims

1. A device for measuring the pressure of a liquid flowing in a tube and extracted from the human body, the device being **characterized in that** it comprises:
- duct-forming means having an axial bore whose wall has an inside face that is substantially flush with the tube, said means including an orifice passing through the wall and opening out in the inside face, a first end connected to a first portion of said tube and a second end connected to a second portion of the tube; and
- a pressure sensor for measuring the pressure of said liquid in the tube, said sensor having a surface which is subjected to the pressure of said liquid, at least a portion of said sensor being mounted in sealed manner in said orifice, in such a manner that said sensitive surface is substantially flush with the inside face of the wall of the duct-forming means.

2. A device according to claim 1, **characterized in that** the dead volume defined by the sensitive surface of the sensor and by the surface in which the inside face of the wall of the duct-forming means is inscribed is less than 10 mm³.

3. A device according to claim 2, **characterized in that** said pressure sensor comprises a body external to the duct-forming means and an extension in the form of a cylindrical skirt suitable for co-operating in sealed manner with said orifice, the pressure sensitive surface being disposed at the inside end of the skirt, the skirt having an axial length that is substantially equal to the thickness of the wall of the duct-forming means.

4. A device according to any one of claims 1 to 3, **characterized in that** said pressure sensitive surface is constituted by an incompressible material suitable for transmitting the pressure which is applied to said surface to the converter element of the sensor.

5. The use of a device according to any one of claims 1 to 4 in making phaco-emulsification apparatus, **characterized in that** one of the tube portions is connected to a suction pump and the other tube portion is connected to a cannula.
